# FASCICULE DE BREVET EUROPEEN

(11) **EP 3 253 330 B1**
(45) Date de publication et mention de la délivrance du brevet: **04.12.2019**
(21) Numéro de dépôt: 15702760.8
(22) Date de dépôt: 04.02.2015
(51) Int. Cl.: A61F 2/24, A61F 2/07, A61F 2/90

(54) **ENDOPROTHESE LUMINALE**
LUMINALE ENDOPROTHESE
LUMINAL ENDOPROSTHESIS

(43) Date de publication de la demande: 13.12.2017
(73) Titulaire: Despalle De Béarn, Olivier, 1040 Bruxelles (BE)
(72) Inventeur: Despalle De Béarn, Olivier, 1040 Bruxelles (BE)
(74) Mandataire: ABYOO
(86) Numéro de dépôt international: PCT/EP2015/052305
(87) Numéro de publication internationale: WO 2016/124235

(56) Documents cités:
- WO-A1-97/40779
- WO-A1-97/40779
- WO-A1-2014/159746
- WO-A1-2014/159746
- WO-A2-2011/143263
- WO-A2-2011/143263
- US-A- 5 741 324
- US-A- 5 741 324
- US-A1- 2007 162 104
- US-A1- 2013 073 026
- US-A1- 2013 073 026

## Description

### Domaine technique

La présente invention concerne une endoprothèse luminale régulatrice de flux comprenant un premier tronçon sensiblement cylindrique et un deuxième tronçon sensiblement cylindrique.

### Etat antérieur de la technique

Une endoprothèse luminale est un dispositif médical destiné à être implantée à l'intérieur de la lumière d'un organe (c'est-à-dire dans le lumen d'un organe).

Une endoprothèse luminale est divulguée dans le document EP1769774A1. Cette endoprothèse comporte une armature cylindrique en fils métalliques entrelacés à l'aide d'une technique de tressage décrite dans le document EP1942832B1.

Malheureusement, une telle endoprothèse a pour inconvénient que les extrémités des fils métalliques la formant sont libres aux deux extrémités de l'endoprothèse. Ces extrémités libres de fils métalliques forment des éléments qui griffent la paroi de l'organe dans lequel l'endoprothèse est insérée, ce qui aboutit à des lésions de cette paroi, notamment à l'extrémité proximale de l'endoprothèse. Ce phénomène est particulièrement marqué lorsque l'endoprothèse est utilisée dans une artère à cause des mouvements de l'artère sous l'effet des pulsations provoquées par le flux sanguin et dues aux battements du cœur. Ces lésions peuvent entrainer des perforations, des lacérations, des dissections ou des ruptures de la paroi de l'organe dans lequel l'endoprothèse est insérée, ce qui entraine de sérieux problèmes médicaux.

La demande internationale WO 2013/043526 A1 concerne un dispositif vasculaire comprenant une structure tubulaire et une structure occlusive. Ladite structure tubulaire comporte des couches interne et externe entre lesquelles est située ladite structure occlusive.

### Divulgation de l'invention

L'invention propose à cet effet une endoprothèse luminale régulatrice de flux comprenant au moins un premier tronçon comportant un premier maillage tressé et un deuxième tronçon comportant un deuxième maillage tressé, dans laquelle à une extrémité de l'endoprothèse, un des tronçons est retourné par rapport à l'autre tronçon de manière à former une endoprothèse comprenant deux tronçons sensiblement coaxiaux et une partie de retournement joignant les deux tronçons, ladite partie de retournement comportant un maillage tressé et étant située à une extrémité de l'endoprothèse, ledit premier maillage tressé et ledit deuxième maillage tressé étant superposés et décalés, ladite endoprothèse étant caractérisée en ce que ledit premier tronçon comportant ledit premier maillage tressé et ledit deuxième tronçon comportant ledit deuxième maillage tressé sont distants de 0,5 à 4 mm.

Le retournement d'un tronçon par rapport à l'autre à une extrémité de l'endoprothèse permet que cette extrémité ne comporte pas d'élément ayant une extrémité libre risquant d'occasionner des lésions de la paroi de l'organe dans lequel l'endoprothèse est insérée. En effet, dès lors qu'un tronçon est retourné par rapport à l'autre, les éléments des tronçons ayant des extrémités acérées, par exemples des fils métalliques, se prolongent d'un tronçon à l'autre et ne comportent pas, à cette extrémité de l'endoprothèse, d'extrémité libre risquant de griffer la paroi de l'organe dans lequel l'endoprothèse est insérée.

L'endoprothèse selon l'invention peut comprendre une ou plusieurs des caractéristiques suivantes, prises isolément ou en combinaison les unes avec les autres :
- le premier tronçon est retourné à l'intérieur du deuxième tronçon,
- le premier tronçon est retourné à l'extérieur du deuxième tronçon,
- les premier et deuxième tronçons et la partie de retournement comportent un maillage,
- le maillage est tressé,
- le maillage donne lieu à des mailles rhombiques,
- les mailles rhombiques ont au moins un angle compris entre 130º et 160º,
- les mailles rhombiques ont une petite diagonale qui est sensiblement parallèle à un axe des tronçons,
- l'endoprothèse comprend au moins un fils métallique,
- le fil métallique comprend un alliage de cobalt/chrome/nickel ou un alliage de nickel/titane,
- les tronçons comportent un matériau dont le module d'élasticité est compris entre 0.050 N/m² et 1N/m²,
- les tronçons sont distants de 0.5 mm à 4 mm,
- l'endoprothèse comporte en outre un moyen d'augmentation de la résistance à l'écrasement radial disposé entre le premier et le deuxième tronçons,
- l'endoprothèse comporte en outre au moins une valve fixée à la partie de retournement, et
- les tronçons sont sensiblement cylindriques et ont des sections sensiblement circulaires.

La présente invention concerne également un procédé de fabrication d'une endoprothèse luminale régulatrice de flux comprenant un premier et un deuxième tronçons sensiblement coaxiaux et une partie de retournement joignant les deux tronçons, le procédé comprenant les étapes suivantes:
- fabrication du premier tronçon,
- fabrication de la partie de retournement, et
- fabrication du deuxième tronçon.

Le procédé selon l'invention peut comprendre une ou plusieurs des caractéristiques suivantes, prises isolément ou en combinaison les unes avec les autres :
- un passage du premier tronçon qui est un tronçon intérieur, dans la lumière d'une bague,
- la fabrication du premier tronçon, la fabrication de la partie de retournement et la fabrication du deuxième tronçon impliquent un tressage,
- le tressage du premier tronçon, le tressage de la partie de retournement et le tressage du deuxième tronçon sont réalisés avec les mêmes fils,
- les fils comprennent un matériau métallique,
- le tressage du premier tronçon est effectué par des fils qui passent à l'extérieur de la bague et le tressage du deuxième tronçon est effectué par des fils qui passent de l'extérieur à l'intérieur de la bague,
- un tube entoure le premier tronçon intérieur lors de la fabrication du deuxième tronçon,
- un moulage de l'endoprothèse afin de créer une endoprothèse à canaux multiples en joignant des segments opposés l'un à l'autre le long de la paroi de l'endoprothèse,
- une couture des segments joints, et
- un insertion d'au moins une endoprothèse dans l'endoprothèse à canaux multiples.

### Brève description des figures

D'autres caractéristiques et avantages de l'invention apparaîtront à la lecture de la description détaillée qui suit pour la compréhension de laquelle on se reportera aux figures annexées parmi lesquelles :
- la figure 1 montre un gros plan d'un maillage selon une réalisation de l'invention;
- la figure 2 montre un tronçon selon une réalisation de l'invention;
- la figure 3 représente une partie d'une endoprothèse selon l'invention comprenant une extrémité proximale de l'endoprothèse selon l'invention;
- la figure 4 est une vue en coupe d'une partie de l'endoprothèse selon l'invention comprenant l'extrémité proximale de l'endoprothèse;
- la figure 5 représente une endoprothèse selon l'invention dans une artère avec un anévrisme;
- la figure 6 représente une variante de l'endoprothèse selon l'invention dans une artère avec un anévrisme;
- la figure 7 représente l'endoprothèse selon l'invention dans une artère avec une dissection;
- la figure 8 représente une endoprothèse 80 à canaux multiples selon une réalisation de l'invention;
- la figure 9 représente une coupe selon un plan perpendiculaire à l'axe de l'endoprothèse à canaux multiples selon l'invention;
- la figure 10 représente une coupe selon un autre plan perpendiculaire à l'axe de l'endoprothèse à canaux multiples selon l'invention;
- la figure 11 représente une variante de l'endoprothèse 80 à canaux multiples selon l'invention;
- les figures 12a, 12b, 12c, 12d, 12e et 12f représentent les étapes d'un procédé de fabrication de l'endoprothèse selon l'invention;
- la figure 13 représente un crochet pour fixer un élément à l'endoprothèse selon l'invention;
- la figure 14 schématise une pièce inférieure d'un moule servant à fabriquer une endoprothèse à canaux multiples selon l'invention;
- la figure 15 schématise une coupe de la pièce inférieure du moule servant à fabriquer une endoprothèse à canaux multiples selon l'invention;
- la figure 16 schématise une autre coupe de la pièce inférieure du moule servant à fabriquer une endoprothèse à canaux multiples selon l'invention; et
- la figure 17 présente une endoprothèse en Y selon une réalisation de la présente invention et un procédé selon la présente invention de fabrication de l'endoprothèse en Y.

### Modes de réalisation de l'invention

La présente invention est décrite avec des réalisations particulières et des références à des figures mais l'invention n'est pas limitée par celles-ci. Les dessins ou figures décrits ne sont que schématiques et ne sont pas limitants. Dans les dessins, certains éléments ne sont pas à l'échelle.

Sur les figures, les éléments identiques ou analogues peuvent porter les mêmes références.

Dans le présent document, l'adjectif « proximal » se réfère à la partie d'un élément en amont par rapport à un flux. Par exemple, dans une artère, une partie proximale d'un élément est la partie la plus proche du cœur car c'est la partie en amont par rapport au flux de sang.

Dans le présent document, l'adjectif « distal » se réfère à la partie d'un élément en aval par rapport à un flux. Par exemple, dans une artère, une partie distale d'un élément est la partie la plus éloignée du cœur car c'est la partie en aval par rapport au flux de sang.

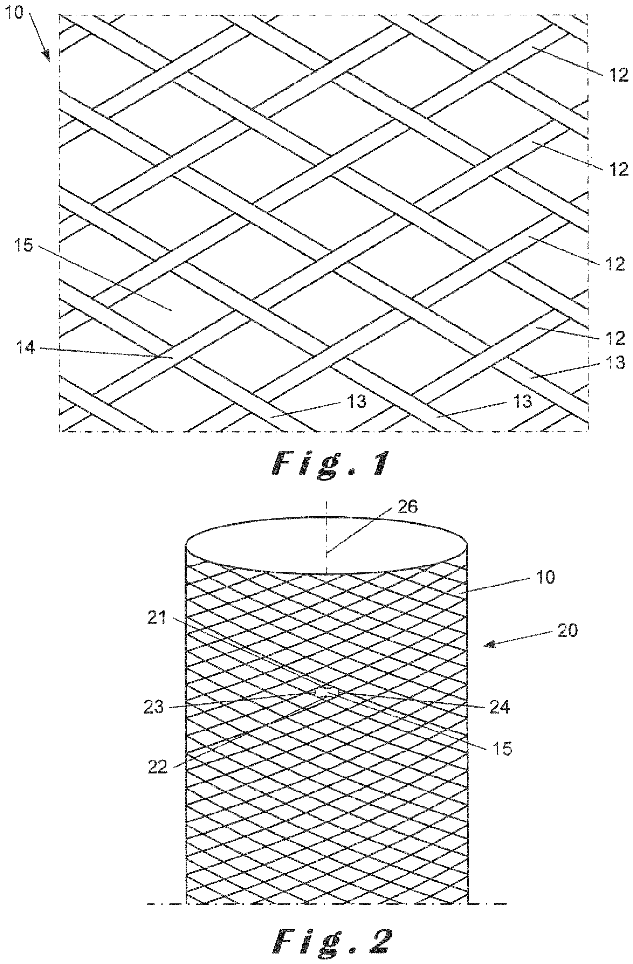

La figure 1 montre un gros plan d'un maillage 10 selon une réalisation de l'invention. Des fils 12 sensiblement parallèles ayant une direction allant de bas-gauche à haut-droite s'entrecroisent avec des fils 13 sensiblement parallèles ayant une direction allant de haut-gauche à bas-droite comme montré à la figure 1, mais d'autres façons de tresser les fils sont possibles sans départir du cadre de l'invention. Un fil 12 passe en alternance au-dessus et en-dessous des fils 13 et un fil 13 passe en alternance au-dessus et en-dessous des fils 12. Le maillage 10, parfois appelé trame, réalisé par les fils 12, 13 est tel qu'une maille 15 formée de deux morceaux de fils 12 et de deux morceaux de fils 13 a sensiblement une forme de losange, autrement dit, la maille 15 est préférentiellement rhombique. D'autres formes sont possibles pour la maille 15, par exemple, une forme de carré (qui est un type particulier de losange), une forme de rectangle, de parallélogramme, de triangle, de pentagone, hexagone, ou n'importe quel polygone.

Dans une réalisation de l'invention, aux endroits où un fil 12 passe au-dessus ou en-dessous d'un fil 13, c'est-à-dire aux croisements 14 du maillage 10, les fils 12, 13 adhèrent l'un à l'autre. Dans une autre réalisation de l'invention, les fils 12, 13 n'adhèrent pas l'un à l'autre aux croisements 14.

Les fils 12, 13 sont préférentiellement dans un matériau biocompatible et non-ferromagnétiques. Les fils 12, 13 comportent préférentiellement un matériau métallique. Les fils 12, 13 sont préférentiellement en un alliage de cobalt/chrome/nickel (Co/Cr/Ni) comme le phynox (marque de la société Aperam Alloys Imphy), le phynox étant aussi appelé aussi Elgiloy (marque de Elgiloy Specialty Metals, American Gage & Machine Company, American Gage & Machine Company Corporation ILLINOIS et COMBINED METALS OF CHICAGO), ou en un métal à mémoire de forme comme un alliage nickel/titane (Ni/Ti) comme le nitinol. Les fils 12, 13 peuvent aussi être faits d'un polymère bio-compatible, comporter des fils de métal recouverts de matériau bio-compatible ou comporter un polymère bio-compatible.

Dans une réalisation préférée de l'invention les fils 12, 13 sont faits dans le même matériau, mais ils peuvent être faits de matériaux différents sans départir du cadre de l'invention. Par exemple, deux fils sur trois peuvent être faits de phynox et un fil sur trois peut être fait de nitinol pour améliorer la mémoire de forme par rapport à une endoprothèse totalement en phynox. Un autre exemple est une endoprothèse où un fil sur cinq est fait d'or pour améliorer la radio-opacité de l'endoprothèse, ce qui permet qu'elle soit aisément détectée à la radiographie.

Les fils 12, 13 sont préférentiellement dans un matériau légèrement élastique. Le module d'élasticité du matériau des fils 12, 13 est préférentiellement entre 0.050 N/m² et 1/m² (entre 50 kN/mm² et 1000 kN/mm²), plus préférentiellement entre 0.100 N/m² et 0.500 N/m², encore plus préférentiellement aux environs de 0.200 N/m² (c'est-à-dire 200 kN/mm²).

Les fils 12, 13 ont préférentiellement un diamètre entre 0.01 µm et 350 µm. Les fils 12, 13 ont plus préférentiellement un diamètre entre 10 µm et 220 µm. Les fils 12, 13 ont encore plus préférentiellement un diamètre de 120 µm, 150 µm ou 180 µm.

Le maillage 10 est préférentiellement tel que sa surface a une porosité de 60 à 75%. Le maillage 10 est plus préférentiellement tel que sa surface a une porosité proche de 70%. La porosité peut être modifiée par une modification du nombre de fils ou par une modification des angles des mailles rhombiques 15.

La figure 2 montre un tronçon 20 selon une réalisation de l'invention. Le tronçon 20 est creux, essentiellement de forme cylindrique avec une section circulaire et a un axe de révolution 26. Le tronçon 20 est préférentiellement un assemblage tressé de fils 12, 13 qui s'entrecroisent en une trame simple selon le maillage 10 montré à la figure 1.

Le maillage 10 est préférentiellement tel que la grande diagonale du losange qu'est la maille 15 est sensiblement perpendiculaire à l'axe 26 du tronçon 20 et la petite diagonale du losange qu'est la maille 15 est sensiblement parallèle à l'axe 26 du tronçon 20, mais d'autres inclinaisons du maillage 10 ou d'un autre maillage sont possibles sans départir du cadre de l'invention. Les angles 21 et 22 dont la bissectrice est la petit diagonale du losange sont égaux et préférentiellement compris entre 130º et 160º et plus préférentiellement aux alentours de 145º, et les angles 23 et 24 dont la bissectrice est la grande diagonale du losange sont égaux et préférentiellement compris entre 30º et 50º et plus préférentiellement aux alentours de 35º, de telle façon que des angles consécutifs du losange soient complémentaires. Les mailles 15 ont préférentiellement des côtés compris entre 1 mm et 2 mm.

Le tronçon 20 a préférentiellement un diamètre entre 1mm et 50mm. Les mailles ont préférentiellement des côtés de longueur telle qu'un tour du tronçon 20 est effectué avec entre 30 et 200 lignes de mailles, une ligne de mailles étant un ensemble de mailles qui se suivent et dont les grandes ou les petites diagonales se suivent.

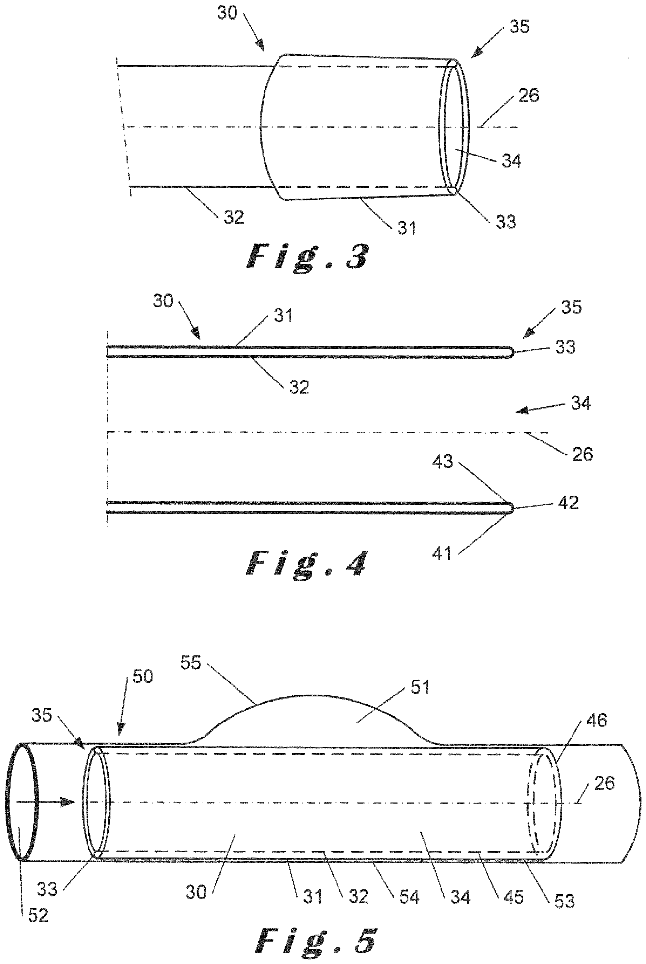

La figure 3 représente une partie d'une endoprothèse 30 selon l'invention comprenant une extrémité proximale 35 de l'endoprothèse 30. L'endoprothèse comprend un tronçon extérieur 31, un tronçon intérieur 32 et une partie de retournement 33. La portion d'espace à l'intérieur du tronçon intérieur 32 est le lumen 34 de l'endoprothèse 30.

Chacun des tronçons intérieur 32 et extérieur 31 est un tronçon 20 tel que représenté à la figure 2. Les tronçons 31, 32 sont sensiblement cylindriques et coaxiaux lorsque l'endoprothèse 30 n'est pas insérée dans un organe et lorsque l'endoprothèse 30 est insérée dans un organe, leur forme s'adapte à l'organe. L'axe 26 déjà présenté à la figure 2 est un axe pour les tronçons 31, 32 et pour l'endoprothèse 30.

Dans l'endoprothèse 30, la superposition des deux tronçons 31, 32 fait que le maillage vu de l'extérieur de l'endoprothèse 30 a un pas en moyenne plus petit que le pas du maillage 10 d'un des tronçons 31, 32 pris séparément comme représenté à la figure 1. Par conséquent, dans l'endoprothèse 30, la superposition des deux tronçons 31, 32 fait que le maillage vu de l'extérieur de l'endoprothèse 30 a une maille en moyenne plus petite que la maille du maillage 10 d'un des tronçons 31, 32 pris séparément comme représenté à la figure 1. C'est la superposition des deux tronçons 31, 32 avec ces mailles de surface en moyenne plus petites qui crée la porosité totale de l'endoprothèse 30.

La figure 4 est une vue en coupe d'une partie de l'endoprothèse 30 selon l'invention comprenant l'extrémité proximale 35 de l'endoprothèse 30, la coupe étant dans un plan passant par l'axe 26. Quand l'endoprothèse 30 n'est pas en place dans un organe, les tronçons 31, 32 sont sensiblement droits dans la direction de l'axe 26 et ont des parois sensiblement parallèles à l'axe 26. Quand l'endoprothèse 30 est en place dans un organe, les tronçons 31, 32 suivent la forment du lumen de l'organe et leur parois ne sont plus nécessairement parallèles. Il arrive que les tronçons 31, 32 se rapprochent l'un de l'autre, notamment dans les endroits où l'organe est courbé.

La partie de retournement 33 fait la jonction entre les tronçons 31, 32. La partie de retournement 31 comprend une première partie 41 dans le prolongement du tronçon extérieur 31 et qui fléchit petit à petit pour joindre une deuxième partie 42 qui est essentiellement dans un plan perpendiculaire à l'axe 26 et une troisième partie 43 dans le prolongement du tronçon intérieur 32 et qui fléchit petit à petit pour joindre la deuxième partie 42.

Dans une réalisation préférée de l'invention, les tronçons intérieur 32 et extérieur 31 sont de longueurs sensiblement égales de façon à ce que le tronçon extérieur 31 recouvre complètement le tronçon intérieur 32. Cependant, dans des réalisations alternatives de l'invention, le tronçon extérieur 31 peut être plus long ou moins long que le tronçon intérieur 32. Pour des raisons de clarté, la partie représentée du tronçon extérieur 31 est moins longue que la partie représentée du tronçon intérieur 32 à la figure 3.

Les tronçons 31, 32 et la partie de retournement 33 sont préférentiellement faits d'une trame de fils continue, de sorte que la continuité des fils des tronçons 31, 32 n'est pas interrompue, en particulier dans la partie de retournement 33.

Dans l'endoprothèse 30, les fils métalliques se poursuivent entre le tronçon intérieur 32, la partie de retournement 35 et le tronçon extérieur 31. Grâce à cela, la partie distale 34 de l'endoprothèse 30 ne comporte pas d'extrémité libres, c'est-à-dire de bouts, de fils métalliques qui pourraient endommager ou déchirer la paroi de l'organe dans lequel l'endoprothèse 30 est insérée, ces extrémités libres étant pointues et acérées. L'endoprothèse 30 ne présente pas de point de rupture ou de faiblesse par soudure ou découpe des intersections des fils.

Le diamètre de l'endoprothèse 30 est préférentiellement compris entre 1 mm et 50 mm. Le diamètre des fils 12, 13 est préférentiellement choisi en fonction du diamètre de l'endoprothèse 30.

La distance 44 entre les deux tronçons 31, 32 est déterminée par la partie de retournement. La distance 44 est dans un intervalle de 0.5 mm à 4 mm. La distance 44 est préférentiellement de l'ordre de 2mm.

Dans une réalisation préférée de l'invention, les deux tronçons 31, 32 ne bougent pas l'un par rapport à l'autre dans une direction parallèle à l'axe 26, deux mailles se faisant face lors de la fabrication de l'endoprothèse se feront toujours face.

La portion d'espace délimitée par les deux tronçons 31, 32 et la partie de retournement 33 est un espace inter-tronçons 45. L'endoprothèse 30 selon l'invention permet d'insérer une structure espace inter-tronçons 45 dans l'espace inter-tronçons 45, par exemple une structure en métal ou nitinol pour augmenter la force radiale de l'endoprothèse 30.

Selon une réalisation de l'invention, à une extrémité distale 46 de l'endoprothèse 30, les extrémités des tronçons 31, 32 sont peu agressives pour la paroi de l'organe dans lequel l'endoprothèse est insérée. Par exemple, les fils 12, 13 des tronçons 31, 32 sont coupés au laser, ce qui forme de petites boules au bout des fils à cause de la chaleur du laser. Les fils 12, 13 des tronçons 31, 32 peuvent être coupés par des moyens mécaniques comme des ciseaux et être traités par électro-polissage pour arrondir chaque extrémité de fil.

Grâce à la structure de tressage des fils 12, 13, l'endoprothèse 30 selon l'invention a une flexibilité suffisante pour suivre la forme des conduits des organes, notamment des vaisseaux sanguins.

La force radiale, c'est-à-dire la résistance à l'écrasement radial, de l'endoprothèse 30 selon l'invention est le résultat du compromis suivant.

D'une part, la force radiale de l'endoprothèse 30 est assez grande pour empêcher l'endoprothèse 30 de se refermer. En particulier, la force radiale de l'endoprothèse 30 est plus grande au niveau de son extrémité proximale 35 que sur le reste de sa structure grâce à la deuxième partie 42 de la partie de retournement 33 qui a une forte composante dans un plan perpendiculaire à l'axe 26, autrement une composante radiale.

D'autre part, la force radiale de l'endoprothèse 30 est suffisamment petite pour permettre au diamètre de l'endoprothèse 30 de devenir minime lorsque l'endoprothèse 30 est étirée, de façon à ce que l'endoprothèse 30 puisse se glisser dans un cathéter pour être insérée dans des organes comme des vaisseaux sanguins.

Le fait que la force radiale de l'endoprothèse 30 est plus grande dans sa partie proximale que dans sa partie distale est particulièrement intéressant dans le cas d'une endoprothèse destinée à être placée dans l'aorte, car le diamètre de l'aorte diminue au fur et à mesure que de l'éloignement au cœur. En effet, l'endoprothèse 30 placée dans l'aorte rétrécit alors son diamètre dans sa partie distale sous la pression de la paroi de l'aorte, tout en conservant un diamètre plus élevé dans sa partie proximale, là où le diamètre de l'aorte est plus grand.

L'endoprothèse 30 selon l'invention présente une bonne fiabilité et un bon maintien de sa forme dans le temps car elle ne présente pas de point de rupture ou de faiblesse par soudure ou découpe intersections des fils et grâce à l'excellente biocompatibilité du matériau choisi (par exemple le phynox)

Dans une réalisation de l'invention, l'endoprothèse 30 ne comporte pas de recouvrement textile. Cependant, l'endoprothèse 30 pourrait en comporter, mais ce mode de réalisation ne ferait pas partie de l'invention.

Dans une réalisation de l'invention, les tronçons 31, 32 sont fixés, par soudure par exemple, l'un à l'autre dans la partie distale de l'endoprothèse, pour éviter que les tronçons ne glissent l'un par rapport à l'autre lors du placement de l'endoprothèse 30.

Dans une réalisation de l'invention, certaines mailles sont fixées au niveau des croisements mais pas toutes ou certains fils sont fixés ensemble au niveau des croisements mais pas tous. Par exemple, les mailles sont fixées uniquement sur une partie proximale de l'endoprothèse mais pas sur une partie distale, ou certains fils sont décollés deux à deux sur une partie distale de l'endoprothèse.

Dans une réalisation de l'invention où les mailles ne sont pas fixées au niveau des croisements, il est possible de faire traverser la paroi l'endoprothèse 30 avec un guide puis un ballon et enfin un stent pour traiter une sténose sur un vaisseau dont l'entrée donne sur l'endoprothèse 30 car les mailles s'écartent pour laisser le passage puis elles reprennent leur place.

Dans une réalisation de l'endoprothèse 30 selon l'invention spécifiquement prévue pour l'aorte, l'endoprothèse 30 a un diamètre d'environ 30mm, une longueur d'environ 150mm, des mailles rhombiques avec une grande diagonale d'environ 2mm et une porosité d'environ 65%.

Même si la présente invention est principalement décrite comme comportant deux tronçons 31, 32 coaxiaux, une endoprothèse comportant plus de deux tronçons et au moins une partie de retournement ne départ pas du cadre de l'invention. Par exemple, une endoprothèse comportant quatre tronçons présenterait l'avantage qu'une extrémité de l'endoprothèse ne comporte pas d'élément ayant une extrémité libre risquant d'occasionner des lésions de la paroi de l'organe dans lequel l'endoprothèse est insérée tout en étant plus rigide que l'endoprothèse ne comportant que deux tronçons.

La figure 5 représente une endoprothèse selon l'invention dans une artère 50 avec un anévrisme 51, qui est une dilatation localisée d'une paroi 54 de l'artère. L'endoprothèse selon l'invention est préférentiellement utilisée dans des vaisseaux sanguins, notamment l'aorte ou dans la boite crânienne, pour des cas d'anévrisme ou de dissection, mais elle peut être aussi utilisée pour des cas d'athérosclérose et de sténose. Une endoprothèse selon l'invention peut aussi être utilisée dans des veines. Une endoprothèse selon l'invention peut aussi être utilisée dans d'autres organes que des vaisseaux sanguins présentant un lumen tel que l'intestin.

Une endoprothèse telle que celle de l'invention est parfois appelée stent, stent graft ou stent pour vaisseau sanguin. L'endoprothèse selon l'invention est qualifiée "régulatrice de flux " de part son action de régulation sur la dynamique du flux du liquide passant dans le lumen de l'organe dans lequel l'endoprothèse est insérée.

Le flux du sang est représenté par une flèche 52 à la figure 5. L'endoprothèse 30 est placée de façon à ce que la partie de retournement 33 soit du côté d'où le sang provient, c'est-à-dire du côté proximal suivant la convention du présent document.

Le sang passe principalement dans le lumen 34 de l'endoprothèse 30 mais aussi dans l'espace 45 inter-tronçons et dans un espace 53 situé entre l'endoprothèse 30 et la paroi 54 de l'artère.

Grâce à la disposition des fils 12, 13 du tronçon extérieur 31 par rapport aux fils 12, 13 du tronçon intérieur 32, autrement dit grâce à la superposition de deux maillages décalés et distant d'une distance 44, l'endoprothèse 30 modifie la dynamique du sang de telle façon que le sang circulant dans l'espace 53 est dirigé en direction de l'axe 26 et que le flux de sang est accéléré dans le lumen 34. L'endoprothèse 30 a donc pour effet que la circulation du sang est meilleure dans l'artère en présence de l'endoprothèse 30 qu'en l'absence de l'endoprothèse 30.

L'endoprothèse 30 selon l'invention permet de réguler le flux du sang en modifiant la dynamique du sang pénétrant dans l'anévrisme 51. En effet, en l'absence de l'endoprothèse 30 selon l'invention, le flux sanguin pénétrant dans l'anévrisme 51 est essentiellement un flux turbulent présentant des vortex. La présence de l'endoprothèse 30 selon l'invention permet que le flux sanguin pénétrant dans l'anévrisme 51 soit laminaire. Cela a plusieurs effets positifs.

D'une part, le flux laminaire au lieu de turbulent réduit la pression sanguine dans l'anévrisme et sur une paroi 55 de l'anévrisme 51. Cette paroi 55 est fragilisée à cause de l'anévrisme et une rupture d'anévrisme peut être fatale pour l'organisme. Réduire la pression subie par la paroi 55 réduit donc la probabilité d'une issue mortelle à l'anévrisme 51. Utiliser une endoprothèse couverte ne permet pas cet effet car il est connu qu'une endoprothèse couverte d'un textile transmet environ 70% de l'onde de pression à la paroi 55 de la partie de l'anévrisme.

D'autre part, un flux turbulent dans l'anévrisme 51 crée dans celui-ci des thromboses, c'est-à-dire des tissus créés par coagulation sanguine, désordonnées qui transmettent la pression sanguine à la paroi 55, alors qu'un flux laminaire crée dans l'anévrisme 51 des thromboses en couches organisées qui ont un effet d'amortissement de la pression. Les thromboses en couches réduisent ainsi la pression exercée par le sang sur la paroi 55 et par conséquent réduisent le risque d'une issue fatale à l'anévrisme 51.

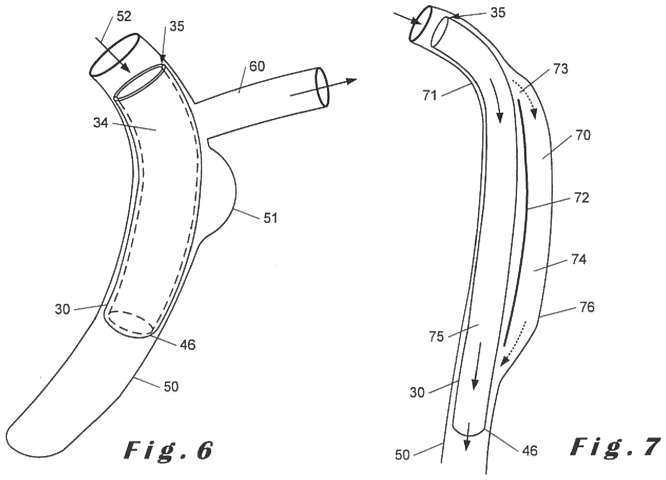

La figure 6 représente une endoprothèse 30 selon l'invention dans une artère 50 avec un anévrisme 51. L'artère 50 représentée à la figure 6 a un tracé relativement sinueux auquel la forme de l'endoprothèse 30 s'adapte. L'excellente adaptabilité de l'endoprothèse 30 à la forme de l'artère est notamment due au tressage de l'endoprothèse 30. Ce tressage donne de plus lieu à un faible risque d'entortillement ou blocage du lumen de l'endoprothèse 30 lors d'une torsion de l'endoprothèse 30, car les fils de l'endoprothèse 30 peuvent glisser les uns sur les autres pour compenser la torsion.

L'artère 50 représentée à la figure 6 est en outre pourvue d'une branche 60 latérale à proximité de l'anévrisme 51. Une endoprothèse dont les parois empêcheraient le sang de passer dans la branche 60 causerait une obturation de la branche 60, avec un risque de thrombose partielle ou occlusion de cette branche. Pour des branches lombaires ou dorsales, cela engendre un risque de paralysie complète ou partielle de l'organisme. Ce problème est courant pour un anévrisme de l'aorte proche des artères rénales. Le placement d'une endoprothèse dont les parois sont hermétiques bloque le passage du sang vers une ou deux des artères rénales.

A contrario, l'endoprothèse 30 selon l'invention permet au sang présent dans son lumen 34 de passer dans la branche 60 car les tronçons 31, 32 de l'endoprothèse 30 ne sont pas hermétiques.

Il est à noter la structure de l'endoprothèse 30 selon l'invention, et en particulier sa force radiale, donne lieu à une bonne apposition des extrémités de l'endoprothèse qui permet d'éviter les fuites de type I.

La figure 7 représente l'endoprothèse 30 selon l'invention dans une artère 50 avec une dissection 70, aussi appelée anévrisme disséquant. La dissection 70 est un décollement de l'intima 72 par rapport aux autres tuniques 76 (media et adventice) de la paroi 71 de l'artère, le sang artériel entrant alors dans l'espace, appelé faux chenal 74, créé par ce décollement.

L'endoprothèse 30 est placée dans le vrai chenal 75, qui est la lumière de l'artère 50. L'endoprothèse 30 maintient ainsi le vrai chenal 75 ouvert et équilibre la pression entre le vrai chenal 75 et le faux chenal 74. L'endoprothèse 30 exerce aussi une poussée radiale dirigée vers l'extérieur qui tend à réduire le diamètre du faux chenal 74 en repoussant l'intima 72 vers le media de l'artère et à comprimer une ou des portes d'entrée 73 de la dissection 70. La poussée exercée par l'endoprothèse 30 est particulièrement grande à l'extrémité proximale 35 de l'endoprothèse 30 grâce à la deuxième partie 42 de la partie de retournement 33 qui a une forte composante radiale. Comme l'endoprothèse 30 est placée de façon telle qu'elle recouvre la porte d'entrée avec l'extrémité proximale 35 de l'endoprothèse 30 proche de la porte d'entrée 73 de la dissection 70, cette poussée particulièrement grande permet de diminuer la taille de la porte d'entrée 73, voire de refermer la porte d'entrée 73 et ainsi de réduire la quantité de sang qui entre dans le faux chenal 74. Cette réduction de la quantité de sang qui entre dans le faux chenal 74 permet de réduire les risques, notamment les risques vitaux, conséquents à la dissection 70.

Le fait que l'extrémité proximale 35 de l'endoprothèse 30 ne comporte pas d'élément ayant une extrémité libre risquant de griffer la paroi de l'organe dans lequel l'endoprothèse est insérée, grâce au retournement d'un deuxième tronçon à l'intérieur ou à l'extérieur d'un premier tronçon, est particulièrement pertinent dans le cas d'une dissection, en particulier de l'aorte, car une déchirure de la membrane intima 72 entre le vrai chenal 75 et le faux chenal 74 est un risque mortel. L'extrémité distale 46 de l'endoprothèse 30 n'étant pas en contact avec la membrane intima 72 entre le vrai chenal 75 et le faux chenal 74, il n'y a pas de risque aussi important dû au fait que l'extrémité distale 46 de l'endoprothèse 30 puisse endommager la paroi de l'organe dans lequel l'endoprothèse 30 est insérée.

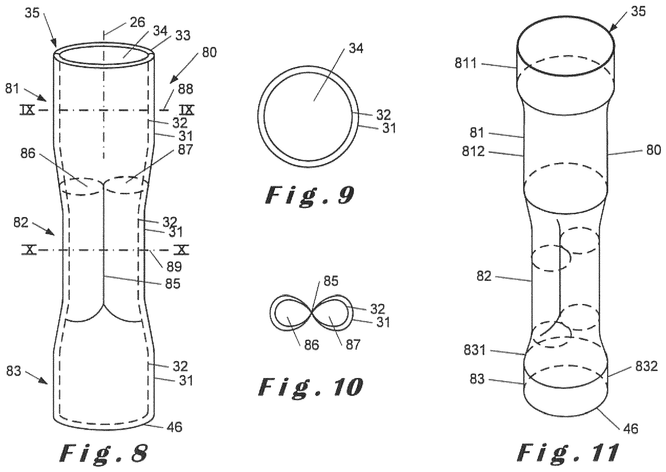

La figure 8 représente une endoprothèse 80 à canaux multiples selon une réalisation de l'invention. La figure 9 représente une coupe selon un plan 88 perpendiculaire à l'axe 26 de l'endoprothèse 80 à canaux multiples, ce plan portant une référence IX à la figure 8. La figure 10 représente une coupe selon un plan 89 perpendiculaire à l'axe 26 de l'endoprothèse 80 à canaux multiples, ce plan portant une référence X à la figure 8. L'endoprothèse 80 à canaux multiples comprend trois parties: une première partie 81 ayant une section de forme sensiblement circulaire (figure 9), une deuxième partie 82 ayant une section sensiblement en forme de 8 (figure 10) et une troisième partie 83 ayant une section de forme sensiblement t circulaire (figure 9).

La première partie 81 est similaire à une partie d'une endoprothèse 30 (figure 3) selon l'invention comprenant l'extrémité proximale 35 de l'endoprothèse 30. La première partie 81 comprend une partie du tronçon intérieur 32, une partie du tronçon extérieur 31 et la partie de retournement 33 de l'endoprothèse 80 à canaux multiples. La troisième partie 83 est similaire à une partie d'une endoprothèse 30 (figure 3) selon l'invention comprenant l'extrémité distale 46 de l'endoprothèse 30. La troisième partie 83 comprend une partie du tronçon intérieur 32 et une partie du tronçon extérieur 31.

La deuxième partie 82 est similaire à une partie médiane d'une endoprothèse 30 (figure 3) selon l'invention ne comprenant ni l'extrémité proximale 35 de l'endoprothèse 30, ni l'extrémité distale 46 de l'endoprothèse 30 et dans laquelle des segments parallèles à l'axe 26 et opposés l'un à l'autre le long de la paroi de l'endoprothèse 30 auraient été joints le long d'une couture 85. La deuxième partie 82 a deux lumens 86, 87. La deuxième partie 82 comprend une partie du tronçon intérieur 32 et une partie du tronçon extérieur 31, les tronçons intérieur 32 et extérieur 31 se rejoignant le long de la couture 85.

Dans une réalisation alternative de l'endoprothèse 80 à canaux multiples la troisième partie 83 n'est pas présente.

La figure 11 représente une variante de l'endoprothèse 80 à canaux multiples. Dans cette variante, la première partie 81 de l'endoprothèse à canaux multiples a elle-même une première partie 811 et une deuxième partie 812. La première partie 811 a un diamètre plus grand que la deuxième partie 812. Dans cette variante, la troisième partie 83 de l'endoprothèse à canaux multiples a elle-même une première partie 831 et une deuxième partie 832. La première partie 811 a un diamètre plus petit que la deuxième partie 812.

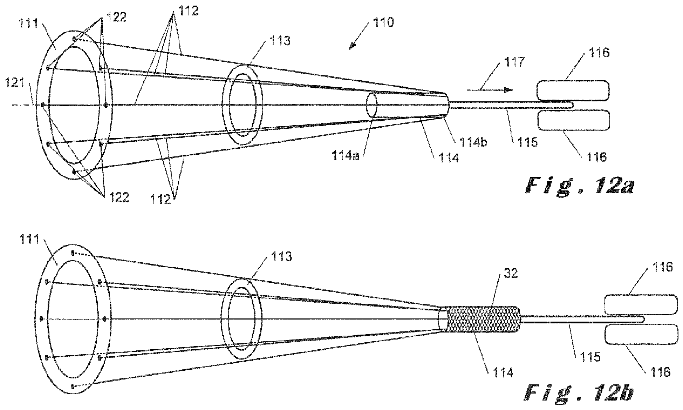

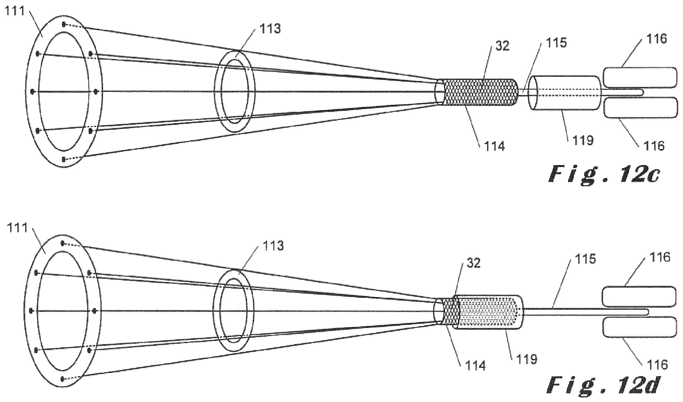

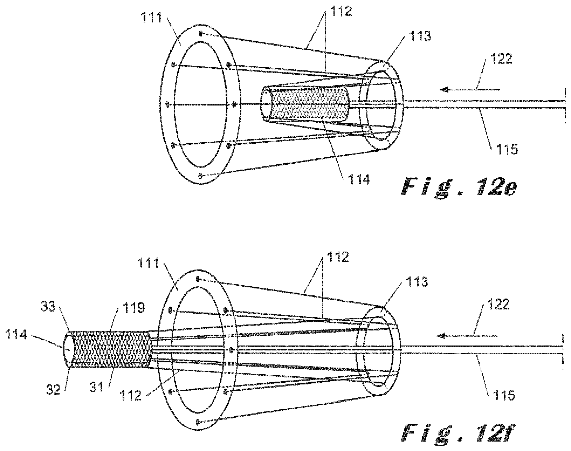

La figure 12 représente un procédé de fabrication de l'endoprothèse 30 selon l'invention. Les éléments représentés à la figure 12 sont les suivants:
- un anneau 111,
- des fils 112,
- une bague 113,
- un mandrin 114,
- une extrémité proximale 114a du mandrin 114,
- une extrémité distale 114b du mandrin 114,
- un guide 115,
- des chenilles 116,
- une première direction 117,
- un tube 119,
- un axe 121, et
- une seconde direction 122.

Pour des raisons de clarté, les éléments de la figure 12 ne sont pas représentés à l'échelle et leur distance respective n'est pas non plus à l'échelle.

L'anneau 111 est arrangé pour tourner autour de l'axe 121. L'anneau 111 peut comporter une pluralité d'anneaux coaxiaux prévus pour tourner dans le même sens ou dans des sens opposés. L'anneau 111 comporte des navettes de tissage 122 qui sur lesquelles les fils 112 sont placés au début du procédé. Les navettes de tissage 122 sont généralement au nombre de 148. Le nombre de navettes de tissage 122 peut varier, notamment en fonction du diamètre choisi pour l'endoprothèse. Pour éviter d'encombrer la figure 12, celle ne représente que le départ des fils 112 par des points.

Les fils 112 sont les fils 12, 13 qui forment le maillage 10 des tronçons 31, 32 de l'endoprothèse 30. Il y a préférentiellement entre 50 et 100 fils 112. Il y a, plus préférentiellement, 78 ou 80 fils. Pour des raisons de clarté, seuls certains des fils 112 sont représentés.

La bague 113 a un diamètre supérieur au diamètre extérieur du tube 119, de façon à ce que le tube 119 puisse passer à l'intérieur de la bague 113.

Le mandrin 114 a un diamètre extérieur égal au diamètre du tronçon intérieur 32 de l'endoprothèse 30. L'extrémité proximale 114a du mandrin correspond à l'extrémité proximale 35 de l'endoprothèse 30. L'extrémité distale 114b du mandrin correspond à l'extrémité distale 46 de l'endoprothèse 30.

Le guide 115 est fixé au mandrin 114 et comporte des moyens pour que les chenilles 116 puissent déplacer le guide 115 (et par conséquent le mandrin 114) le long de l'axe 121.

Les chenilles 116 déplacent le guide 115 dans les deux directions le long de l'axe 121. Les chenilles 116 sont préférentiellement au nombre de deux.

La première direction 117 est la première direction de mouvement du mandrin par rapport à l'anneau 111 et à la bague 113.

Le tube 119 est creux. Son diamètre intérieur est tel que le mandrin 114 avec le tronçon intérieur 32 peuvent être insérés à l'intérieur du tube 119. Son diamètre extérieur est égal au diamètre du tronçon extérieur 31. L'épaisseur du tube 119 est la distance 44 entre le tronçon intérieur 32 et le tronçon extérieur 31. Le tube 119 est préférentiellement de même longueur que le mandrin 114.

L'axe 121 est l'axe de l'anneau 11, de la bague 113, du mandrin 114, du guide 115 et du tube 119.

La seconde direction 122 est la seconde direction de mouvement du mandrin par rapport à l'anneau 111 et à la bague 113.

La figure 12a représente le procédé selon l'invention au moment du début du tissage. Les fils 112 partent de l'anneau 111, passent autour de la bague 113, passent autour du mandrin 114 et sont attachés à l'extrémité distale 114b du mandrin. Dans une réalisation de l'invention, les fils 112 auront préalablement été coupés au laser, ce qui forme de petites boules au bout des fils à cause de la chaleur du laser.

Les chenilles 116 déplacent le guide 115 et le mandrin 114 dans la première direction 117, c'est-à-dire vers la droite sur la figure 12. En même temps, les anneaux de l'anneau 111 tournent de façon à former le tissage 10 pour le tronçon intérieur 32 autour du mandrin 114.

La bague 113 peut aussi permettre de garder un angle de tressage déterminé par rapport à l'axe du système.

La figure 12b représente le procédé selon l'invention après le tissage du tronçon intérieur 32 sur le mandrin 114. Le mouvement du guide 115 et du mandrin 114 s'arrête à ce moment ainsi que le mouvement de l'anneau 111.

Les figures 12c et 12d représentent l'étape suivante du procédé selon l'invention. Le tube 119 est inséré autour du tronçon intérieur 32 qui est lui-même autour du mandrin 114 de façon à ce que l'extrémité du tube 119 soit à la même hauteur que l'extrémité proximale 114a du mandrin 114. Le tube 119 est ensuite bloqué par rapport au mandrin 114. L'objet du tube 119 est de bloquer les fils 112 à l'extrémité proximale 114a du mandrin pour former la partie de retournement 33 de l'endoprothèse 30 et de fournir un support au tressage du tronçon extérieur. Un autre moyen de bloquer les fils 112 à l'extrémité proximale 114a du mandrin pourrait être envisager sans départir du cadre de l'invention. Par exemple, les fils 112 pourraient être bloqués par des attaches à l'extrémité proximale 114a du mandrin.

Les chenilles 116 déplacent ensuite le guide 115 et le mandrin 114 dans la seconde direction 122, c'est-à-dire vers la gauche sur la figure 12, sans qu'il n'y ait de rotation de l'anneau 11. Ce déplacement amène le mandrin 114 et le tronçon intérieur 32 qui recouvre le mandrin 114 à traverser la lumière de la bague 113. Ce déplacement s'arrête quand les fils 112 sont de nouveau sous tension.

La figure 12e représente le procédé selon l'invention au moment du début du tissage du tronçon extérieur 31. Les chenilles 116 déplacent le guide 115 et le mandrin 114 dans la seconde direction 122. En même temps, les anneaux de l'anneau 111 tournent de façon à former le tissage 10 pour le tronçon extérieur 31 autour du tube 119. Les fils 112 partent de l'anneau 111, passent de l'extérieur à l'intérieur de la bague 113, passent autour du tube 119 et rejoignent l'extrémité proximale du premier tronçon 32 déjà tressé. L'endroit du tressage où les fils 112 changent de direction est la partie de retournement 33 de l'endoprothèse.

La passage du mandrin 114 sur lequel le tronçon intérieur 32 est tressé dans la bague 113 permet le tressage de la partie de retournement 33 et du tronçon extérieur 31 sans déconnection des fils 112. Les mêmes fils 112 sont donc utilisés pour le tronçon intérieur 32, le tronçon extérieur 31 et la partie de retournement 33. La bague 113 permet qu'une tension soit présente dans les fils 112 lors du tressage du tronçon extérieur 31 et permet que le mouvement dans l'anneau 111 soient reflétés dans le mouvement des fils 112 au niveau du tube 119 pour la réalisation du tressage de la partie de retournement 33 et du tronçon extérieur 31. La bague 113 permet aussi que les tressages des tronçons intérieur 32 et extérieur 31 se fassent sous le même angle de tressage par rapport à l'axe 121.

La figure 12f représente le procédé selon l'invention après le tissage du tronçon extérieur 31. Le maillage du tronçon intérieur 32 n'est pas représenté à la figure 12f pour éviter une surcharge du schéma. Le mouvement du guide 115 et du mandrin 114 s'arrête à ce moment ainsi que le mouvement de l'anneau 111.

Les fils 112 sont alors coupés à l'extrémité distale du tronçon extérieur 31, de façon à former l'endoprothèse 30. Ils peuvent être coupés au laser ou d'autre façon.

Le tube 119 est retiré. Le mandrin 114 est retiré.

Par la suite, l'endoprothèse 30 peut subir des étapes supplémentaires de traitement. Par exemple un électro-polissage des fils 112 pour arrondir chaque extrémité de fil, un traitement thermique qui consolide la forme de l'endoprothèse 30 et qui colle les maillages des tronçons intérieur 32 et extérieur 31 ou tout autre étape. Un autre exemple est une étape de décapage de l'endoprothèse 30.

Dans une réalisation de l'invention, l'endoprothèse 30 est formée par le tressage d'un seul tronçon qui est ensuite retourné de façon à former un tronçon intérieur 32, un tronçon extérieur 31 et une partie de retournement 33. Ce retournement, ou retroussement, peut être effectué par des moyens mécaniques ou manuellement.

Après la fabrication de l'endoprothèse 30, d'autre éléments peuvent être attachés à l'endoprothèse, ce qui peut être fait avant le placement de l'endoprothèse 30 dans un organisme ou après le placement placement de l'endoprothèse 30 dans un organisme. Par exemple, une valve de type TAVI (Transcatheter Aortic Valve Implantation en anglais = implantation de valve aortique par transcathéter en français) peut être attachée à l'extrémité proximale 35 de l'endoprothèse 30, par exemple en l'attachant à la partie de retournement 33. La valve et l'endoprothèse 30 sont placées en même temps dans l'organisme, à la jonction du cœur et de l'aorte pour la valve et dans l'aorte pour l'endoprothèse 30. L'endoprothèse selon l'invention permet d'une part une bonne fixation de la valve car la partie de retournement 33 est solidement fixée aux deux tronçons 31, 32 puisqu'elle est faite des mêmes fils 12, 13 que les tronçons 31, 32 et d'autre part, la partie proximale de l'endoprothèse selon l'invention ne risque pas d'endommager la valve puisque la partie proximale de l'endoprothèse ne comporte pas d'extrémité libre de fil agressive pour la valve.

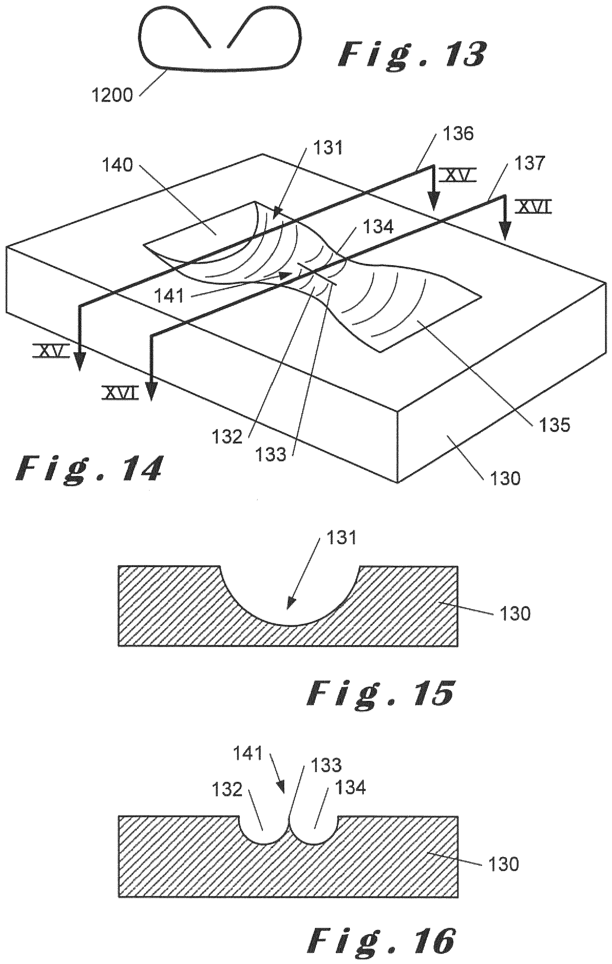

La valve TAVI, ou un autre élément, peut être fixée à l'endoprothèse 30 grâce à des crochets comme celui représenté à la figure 13. Les crochets sont préférentiellement dans le même matériau que l'endoprothèse 30.

L'endoprothèse 80 à canaux multiples selon une réalisation de l'invention peut être fabriquée avec un moule dont une pièce inférieure 130 est schématisée à la figure 14. Une pièce supérieur du moule n'est pas représentée sur la figure car elle est sensiblement identique à la pièce inférieure 130. La pièce inférieure 130 comprend un creux 140. La figure 15 représente une coupe selon un plan 136, ce plan portant une référence XV à la figure 14. La figure 16 représente une coupe selon un plan 137, ce plan portant une référence XVI à la figure 14. Le creux 140 comprend trois parties: une première partie 131 ayant une section en forme de demi-cercle (figure 15), une deuxième partie 141 ayant une section sensiblement en forme de ω (figure 16) et une troisième partie 135 ayant une section en forme de demi-cercle (figure 15). La deuxième partie 141 comprend elle-même deux parties 132, 134 avec une section en forme de demi-cercle, séparées par une arête 133.

Un procédé pour réaliser l'endoprothèse 80 à canaux multiples, à partir d'une endoprothèse 30, est le suivant.

Une endoprothèse 30 ayant une longueur égale ou inférieure à la longueur du creux 140, et un diamètre égal ou inférieur à la largeur du creux 140 est insérée dans le creux 140. La pièce supérieure du moule est placée sur la pièce inférieure 130 de façon à ce que les deux pièces se fassent face et de façon à ce que des segments sensiblement diamétralement opposés de l'endoprothèse 30 et parallèles à l'axe 26 se rejoignent sur l'arête 133 pour former la couture 85 (figure 10). Un traitement thermique est alors préférentiellement appliqué pour consolider la forme de l'endoprothèse 80 à canaux multiples. Ensuite, la pièce supérieure du moule est enlevée et un fil préférentiellement de même matériau que les fils de l'endoprothèse 30 est cousu sur la couture 85 pour fixer ensemble les segments sensiblement diamétralement opposés de l'endoprothèse 30, lors d'une étape de couture.

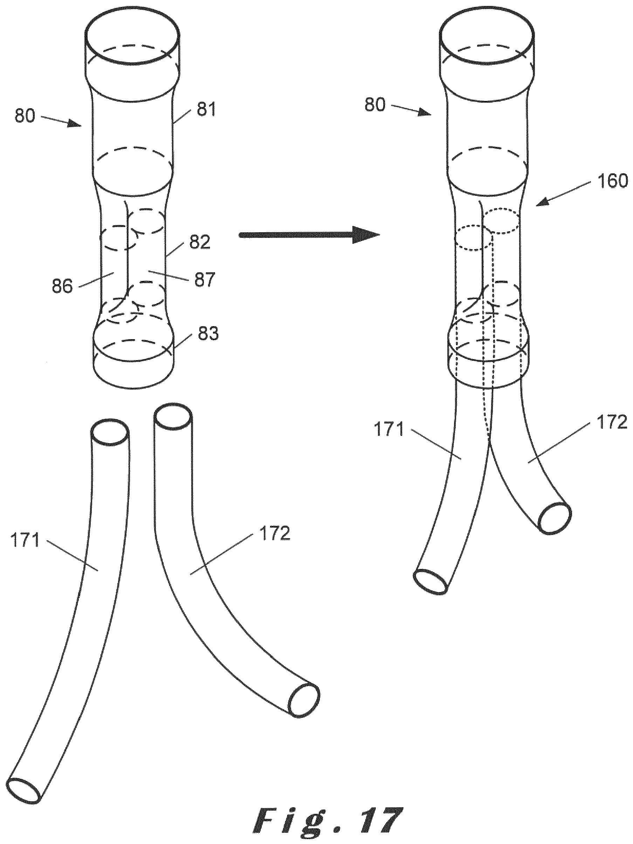

La figure 17 présente une endoprothèse en Y 160 selon une réalisation de la présente invention et un procédé de fabrication de l'endoprothèse en Y 160. Une première endoprothèse 171 et une seconde endoprothèse 172, du type de l'endoprothèse 30 représentée aux figures 3 et 4, sont insérées d'abord dans une troisième partie 83, puis dans une deuxième partie 82 d'une endoprothèse 80 à canaux multiples, de façon à ce que la première endoprothèse 171 soit dans un premier lumen 86 de la deuxième partie 82 et la seconde endoprothèse 172 soit dans un second lumen 87 de la deuxième partie 82. Le diamètre du lumen 86 de la deuxième partie 82 de l'endoprothèse 80 à canaux multiples est supérieur au diamètre extérieur de la première endoprothèse 171 et le diamètre intérieur du second lumen 87 de la deuxième partie 82 de l'endoprothèse 80 à canaux multiples est supérieur au diamètre extérieur de la seconde endoprothèse 172. La première endoprothèse 171 et la seconde endoprothèse 172 sont préférentiellement cousues à l'endoprothèse 80 à canaux multiples. L'endoprothèse en Y 160 est préférentiellement placée au carrefour iliaque.

L'endoprothèse en Y 160 a une forme telle que tout le flot de sang qui arrive du cœur est divisé entre les deux endoprothèses 171, 172.

Même si la figure 17 présente une réalisation de l'invention dans laquelle deux endoprothèses 171, 172 sont insérées dans la troisième partie 83 et la deuxième partie 82 de l'endoprothèse 80 à canaux multiples, des réalisations de l'invention où
- une seule endoprothèse est insérée,
- plus de deux endoprothèses sont insérées,
- l'insertion se fait uniquement dans la troisième partie, ou uniquement dans la deuxième partie notamment si la troisième partie n'est pas présente,
sont possibles sans départir du cadre de la présente invention.

## Revendications

1. Endoprothèse (30) luminale régulatrice de flux comprenant au moins un premier tronçon comportant un premier maillage tressé et un deuxième tronçon comportant un deuxième maillage tressé, dans laquelle à une extrémité (35) de l'endoprothèse, un des tronçons est retourné par rapport à l'autre tronçon de manière à former une endoprothèse (30) comprenant deux tronçons (31, 32) sensiblement coaxiaux et une partie de retournement (33) joignant les deux tronçons (31, 32), ladite partie de retournement (33) comportant un maillage tressé et étant située à une extrémité (35) de l'endoprothèse (30), ledit premier maillage tressé et ledit deuxième maillage tressé étant superposés et décalés, ladite endoprothèse (30) étant **caractérisée en ce que** ledit premier tronçon comportant ledit premier maillage tressé et ledit deuxième tronçon comportant ledit deuxième maillage tressé (31, 32) sont distants de 0,5 à 4 mm.

2. Endoprothèse selon l'une quelconque des revendications précédentes, dans laquelle les tronçons (31, 32) comportent un matériau dont le module d'élasticité est compris entre 0.050 N/m² et 1 N/m².

3. Endoprothèse selon l'une quelconque des revendications précédentes, dans laquelle le maillage a une surface d'une porosité de 60 à 75%, de préférence proche de 70%.

4. Endoprothèse selon l'une quelconque des revendications précédentes, dans laquelle le maillage est tel qu'une superposition des deux tronçons est perméable au sang.

5. Endoprothèse selon l'une quelconque des revendications précédentes, dans laquelle des mailles formant le maillage ont des côtés compris entre 1 mm et 2 mm.

6. Endoprothèse (80) à canaux multiples comprenant une première partie (81) formée d'une endroprothèse selon l'une quelconque des revendications précédentes et une deuxième partie (82) ayant une section sensiblement en forme du numéro huit.

7. Endoprothèse selon la revendication précédente, dans laquelle l'endoprothèse (80) à canaux multiples comprend en outre une troisième partie (83) ayant une section de forme sensiblement circulaire et dans laquelle au moins une endoprothèse (171, 172) est insérée dans la troisième partie (83) de l'endoprothèse (80) à canaux multiples.

8. Endoprothèse selon l'une quelconque des revendications 6 et 7, dans laquelle au moins une endoprothèse (171, 172) est insérée dans la deuxième partie (82) de l'endoprothèse (80) à canaux multiples.

9. Procédé de fabrication d'une endoprothèse (30) selon l'une quelconque des revendications précédentes, le procédé comprenant les étapes suivantes :
- fabrication du premier tronçon (32),
- fabrication de la partie de retournement (33), et
- fabrication du deuxième tronçon (31).

10. Procédé de fabrication d'une endoprothèse (30) selon la revendication précédente, comprenant en outre un passage du premier tronçon qui est un tronçon intérieur (32), dans la lumière d'une bague (113).

11. Procédé selon la revendication 9 ou la revendication 10, dans lequel la fabrication du premier tronçon, la fabrication de la partie de retournement (33) et la fabrication du deuxième tronçon impliquent un tressage et dans lequel le tressage du premier tronçon, le tressage de la partie de retournement et le tressage du deuxième tronçon sont réalisés avec les mêmes fils (112).

12. Procédé selon la revendication précédente, dans lequel le tressage du premier tronçon est effectué par des fils (112) qui passent à l'extérieur de la bague (113) et le tressage du deuxième tronçon (31) est effectué par des fils (112) qui passent de l'extérieur à l'intérieur de la bague (113).

13. Procédé selon l'une quelconque des revendications 9 à 12, dans lequel un tube (119) entoure le premier tronçon intérieur lors de la fabrication du deuxième tronçon.

14. Procédé selon l'une quelconque des revendications 9 à 13 comprenant en outre un moulage de l'endoprothèse (30) afin de créer une endoprothèse (80) à canaux multiples en joignant des segments opposés l'un à l'autre le long de la paroi de l'endoprothèse (30).

15. Procédé selon la revendication précédente, comprenant en outre une couture des segments joints, et comprenant, de préférence, une insertion d'au moins une endoprothèse (30) dans l'endoprothèse (80) à canaux multiples.

## Patentansprüche

1. Den Fluss regulierende luminale Endoprothese (30), umfassend wenigstens einen ersten Abschnitt, der eine erste geflochtene Vernetzung und einen zweiten Abschnitt aufweist, umfassend eine zweite geflochtene Vernetzung, bei der an einem Ende (35) der Endoprothese einer der Abschnitte im Verhältnis zum anderen Abschnitt derart umgekehrt ist, dass eine Endoprothese (30) gebildet ist, umfassend zwei deutlich koaxiale Abschnitte (31, 32) und einen Umkehrteil (33), der die zwei Abschnitte (31, 32) verbindet, wobei der genannte Umkehrteil (33) eine geflochtene Vernetzung aufweist und an einem Ende (35) der Endoprothese (30) angeordnet ist, wobei die genannte erste geflochtene Vernetzung und die genannte zweite geflochtene Vernetzung übereinander gelagert und versetzt sind, wobei die genannte Endoprothese (30) **dadurch gekennzeichnet ist, dass** der erste Abschnitt die genannte geflochtene Vernetzung aufweist und der genannte zweite Abschnitt die genannte zweite geflochtene Vernetzung (31, 32) aufweist, die um 0,5 bis 4 mm entfernt sind.

2. Endoprothese gemäß irgendeinem der voranstehenden Ansprüche, bei der die Abschnitte (31, 32) ein Material aufweisen, dessen Elastizitätsmodul zwischen 0,050 N/m² und 1 N/m² inbegriffen ist.

3. Endoprothese gemäß irgendeinem der voranstehenden Ansprüche, bei der die Vernetzung eine Oberfläche mit einer Porosität von 60 bis 75 %, bevorzugt von ungefähr 70 % aufweist.

4. Endoprothese gemäß irgendeinem der voranstehenden Ansprüche, bei der die Vernetzung derart ist, dass eine Überlagerung von zwei Abschnitten für Blut durchlässig ist.

5. Endoprothese gemäß irgendeinem der voranstehenden Ansprüche, bei der die die Vernetzung bildenden Maschen zwischen 1 mm und 2 mm inbegriffene Seiten aufweisen.

6. Endoprothese (80) mit multiplen Kanälen, umfassend einen ersten Teil (81), der aus einer Endoprothese gemäß irgendeinem der voranstehenden Ansprüche gebildet ist, und einen zweiten Teil (82), der deutlich einen Querschnitt in Form der Nummer acht aufweist.

7. Endoprothese gemäß dem voranstehenden Anspruch, bei dem die Endoprothese (80) mit multiplen Kanälen darüber hinaus einen dritten Teil (83) umfasst, der einen Querschnitt in deutlich kreisförmiger Form aufweist und bei der wenigstens eine Endoprothese (171, 172) in den dritten Teil (83) der Endoprothese (80) mit multiplen Kanälen eingefügt ist.

8. Endoprothese gemäß irgendeinem der Ansprüche 6 und 7, bei der wenigstens eine Endoprothese (171, 172) in den zweiten Teil (82) der Endoprothese (80) mit multiplen Kanälen eingefügt ist.

9. Herstellungsverfahren einer Endoprothese (30) gemäß irgendeinem der voranstehenden Ansprüche, wobei das Verfahren die folgenden Schritte umfasst:
- Herstellung des ersten Abschnitts (32),
- Herstellung des Umkehrteils (33) und
- Herstellung des zweiten Abschnitts (31).

10. Herstellungsverfahren einer Endoprothese (30) gemäß dem voranstehenden Anspruch, umfassend darüber hinaus einen Durchgang des ersten Abschnitts, der ein innerer Abschnitt (32) ist, in der Öffnung eines Rings (113).

11. Verfahren gemäß Anspruch 9 oder Anspruch 10, bei dem die Herstellung des ersten Abschnitts, die Herstellung des Umkehrteils (33) und die Herstellung des zweiten Abschnitts ein Geflecht implizieren und bei dem das Geflecht des ersten Abschnitts, das Geflecht des Umkehrteils und das Geflecht des zweiten Abschnitts mit denselben Drähten (112) realisiert sind.

12. Verfahren gemäß dem voranstehenden Abschnitt, bei dem das Geflecht des ersten Abschnitts durch Drähte (112) durchgeführt ist, die an der Außenseite des Rings (113) vorbeilaufen, und das Geflecht des zweiten Abschnitts (31) durch Drähte (112) durchgeführt ist, die von der Außen- zur Innenseite des Rings (113) verlaufen.

13. Verfahren gemäß irgendeinem der Ansprüche 9 bis 12, bei dem eine Röhre (119) den ersten inneren Abschnitt bei der Herstellung des zweiten Abschnitts umgibt.

14. Verfahren gemäß irgendeinem der Ansprüche 9 bis 13, umfassend darüber hinaus einen Abguss der Endoprothese (30), um eine Endoprothese (80) mit multiplen Kanälen zu schaffen, indem einander entgegengesetzte Segmente entlang der Wand der Endoprothese (30) verbunden werden.

15. Verfahren gemäß dem voranstehenden Anspruch, umfassend darüber hinaus eine Naht der verbundenen Segmente und umfassend bevorzugt ein Einfügen wenigstens einer Endoprothese (30) in die Endoprothese (80) mit multiplen Kanälen.

## Claims

1. A flow-regulating luminal endoprosthesis (30) comprising at least one first section and one second section, with each one of the sections comprising a braided meshing, wherein at an end (35) of the endoprosthesis, one of the sections is turned over with respect to the other section in such a way as to form an endoprosthesis (30) comprising two substantially coaxial sections (31, 32) and a turned over part (33) joining the two sections (31, 32) said turned over part (33) comprising a braided meshing and being located at an end (35) of the endoprosthesis (30), said first and second braided meshing being superimposed and separated, said endoprosthesis (30) being **characterised in that** the said first section comprising the first braided meshing and the second said section comprising the second braided meshing (31,32) are separated by 0.5 mm to 4 mm.

2. The endoprosthesis according to the previous claims, wherein the sections (31, 32) comprise a material the elastic modulus of which is between 0.050 N/m² and 1 N/m².

3. The endoprosthesis according to any one of the previous claims, wherein the meshing has a surface with a porosity of 60 to 75%, preferably close to 70%.

4. The endoprosthesis according to any one of the previous claims, wherein the meshing is such that a superposition of the two sections is permeable to blood.

5. The endoprosthesis according to any one of the previous claims, wherein meshes forming the meshing have sides between 1 mm and 2 mm.

6. A multi-channel endoprosthesis (80) comprising a first portion (81) formed from an endoprosthesis according to any one of the previous claims and a second portion (82) that has a section substantially in the shape of the number eight.

7. The endoprosthesis according to the previous claim, wherein the multi-channel endoprosthesis (80) further comprises a third portion (83) that has a section with a substantially circular shape and wherein at least one endoprosthesis (171, 172) is inserted into the third portion (83) of the multi-channel endoprosthesis (80).

8. The endoprosthesis according to any one of claims 6 and 7, wherein at least one endoprosthesis (171, 172) is inserted into the second portion (82) of the multi-channel endoprosthesis (80).

9. A method for manufacturing a flow-regulating luminal endoprosthesis (30) according to any one of the previous claims, consisting of the following steps:
- Manufacturing of the first section (32),
- Manufacturing of the turned over part (33), and
- Manufacturing of the second section (31).

10. The method for manufacturing an endoprosthesis (30) according to the previous claim, further comprising a passing of the first section which is an inside section (32), in the hole of a ring (113).

11. The method according to claim 9 or claim 10, wherein the manufacture of the first section, the manufacture of the turned over part (33) and the manufacture of the second section entail a braiding and wherein the braiding of the first section, the braiding of the turned over part and the braiding of the second section are carried out with the same wires (112).

12. The method according to the previous claim, wherein the braiding of the first section is carried out by wires (112) which pass outside the ring (113) and the braiding of the second section (31) is carried out by wires (112) which pass from the outside to the inside of the ring (113).

13. The method according to any one of the claims 9 to 12 wherein a tube (119) surrounds the first inside section during the manufacture of the second section.

14. The method according to any one of the claims 9 to 13 further comprising a moulding of the endoprosthesis (30) in order to create a multi-channel endoprosthesis (80) by joining opposite segments to one another along the wall of the endoprosthesis (30).

15. The method according to the previous claim, further comprising a seam of the segments joined, and comprising, preferably, an insertion of at least one endoprosthesis (30) into the multi-channel endoprosthesis (80).
